# EUROPÄISCHE PATENTANMELDUNG

(11) **EP 1 362 566 A1**
(43) Veröffentlichungstag der Anmeldung: **19.11.2003**
(21) Anmeldenummer: 03011238.7
(22) Anmeldetag: 16.05.2003
(51) Int. Cl.: A61F 6/04, B29C 41/14

(54) **Kondom und Verfahren zur Herstellung eines Kondoms**

(30) Priorität: 18.05.2002 DE 10222268
(71) Anmelder: MAPA GmbH, Gummi- und Plastikwerke, D-27404 Zeven (DE)
(72) Erfinder: Marr, Günter, 21224 Rosengarten (DE)
(74) Vertreter: Patentanwälte Hauck, Graalfs, Wehnert, Döring, Siemons, Schildberg

(57) **Zusammenfassung**

Kondom umfassend von innen nach außen
- eine Innenschicht hergestellt aus synthetischem Latex,
- eine Zwischenschicht hergestellt aus Naturkautschuklatex und
- eine Außenschicht hergestellt aus synthetischem Latex.

## Beschreibung

Die Erfindung bezieht sich auf ein Kondom und auf ein Verfahren zur Herstellung eines Kondoms.

Kondome sind aus Latex hergestellte mechanische Mittel zur Kontrazeption, die eine einenends geschlossene und anderenends offene Hülle aus dünnem Kautschuk bzw. Gummi aufweisen, die zum Geschlechtsverkehr über den Penis gestreift wird. Ein Vorteil von Kondomen ist ein gewisser Schutz gegen die Übertragung von Geschlechtskrankheiten, heute insbesondere auch von HIV. Nachteilig ist die Anwendung von Kondomen durch Latexallergiker. Bei Latexallergien können insbesondere Kontaktekzeme entstehen.

Davon ausgehend liegt der Erfindung die Aufgabe zugrunde, ein Kondom zur Verfügung zu stellen, das von Latexallergikern benutzt werden kann, ohne entsprechende allergische Reaktionen hervorzurufen. Ferner soll ein Verfahren zur Herstellung eines solchen Kondoms zur Verfügung gestellt werden.

Die Aufgabe wird durch ein Kondom mit den Merkmalen des Anspruches 1 gelöst. Ferner wird sie durch ein Verfahren zur Herstellung eines Kondoms mit den Merkmalen des Anspruches 10 gelöst. Vorteilhafte Ausgestaltungen sind in den Unteransprüchen angegeben.

Das erfindungsgemäße Kondom umfasst von innen nach außen
- eine Innenschicht hergestellt aus synthetischem Latex,
- eine Zwischenschicht hergestellt aus Naturkautschuklatex und
- eine Außenschicht hergestellt aus synthetischem Latex.

Die Anwendung eines aus synthetischem Latex hergestellten Produktes ruft bei Latexallergikern keine allergischen Reaktionen hervor. Ein Kondom aus synthetischem Latex bedürfte allerdings einer relativ hohen Wandstärke, um die Anforderungen der DIN-Norm EN 600 zu erfüllen. Dies gilt insbesondere für die Festigkeitsanforderungen. Ein solches Kondom wäre in der Herstellung verhältnismäßig aufwändig und hätte aufgrund seiner hohen Wandstärke Anwendungsnachteile. Das erfindungsgemäße Kondom hat eine Sandwichstruktur. Es ist so aufgebaut, dass es sowohl die Festigkeit eines Kondoms aus Naturkautschuklatex hat als auch den Kontakt des Benutzers mit Naturkautschuklatex durch eine Innenschicht und eine Außenschicht hergestellt aus synthetischem Latex verhindert. Genauer gesagt verhindert die durch das synthetische Material bewirkte Proteinundurchlässigkeit der Innenschicht und der Außenschicht, dass der Benutzer mit Latexproteinen aus der Zwischenschicht in Kontakt kommt, welche die Latexallergie hervorrufen. Die Innenschicht und die Außenschicht sind hierfür bevorzugt im Wesentlichen aus synthetischem Latex hergestellt, zumindest jedoch aus einer Zusammensetzung mit einem Gehalt an synthetischem Latex, der die gewünschte Proteinundurchlässigkeit bewirkt. Bevorzugt werden die Innenschicht und die Außenschicht am Rand der Öffnung des Kondoms miteinander verbunden, um die Zwischenschicht vollständig einzuschließen. Es versteht sich, dass die Latices in den verschiedenen Schichten mittels eines Vulkanisators mehr oder weniger vulkanisiert bzw. vernetzt sind. Die Zusammensetzungen der Innenschicht, Zwischenschicht und Außenschicht können so gewählt werden, dass sie ein elastisches Kondom ergeben, das den Anforderungen der Empfängnisverhütung genügt und Schutz vor übertragbaren Krankheiten durch Geschlechtsverkehr bietet. Weiterhin sind die Zusammensetzungen so gewählt, dass sie in Verbindung mit Trenn- bzw.

Gleitmitteln in flüssiger bis fester Form ihre physikalischen Eigenschaften für einen sicheren Geschlechtsverkehr behalten.

Gemäß einer bevorzugten Ausgestaltung durchdringen die Innenschicht und die Zwischenschicht und/oder die Zwischenschicht und die Außenschicht einander im Grenzbereich und/oder sind miteinander im Grenzbereich vernetzt. Hierdurch wird eine Verbindung der verschiedenen Schichten bewirkt, die die Belastbarkeit des Kondoms begünstigt. Gemäß einer weiteren Ausgestaltung enthält auch die Zusammensetzung aus der die Zwischenschicht hergestellt ist, synthetischen Latex, wodurch die Durchdringung und/oder Vernetzung der Schichten im Grenzbereich begünstigt wird. Vorzugsweise handelt es sich um denselben synthetischen Latex, der für die Herstellung der Innenschicht und/oder der Außenschicht verwendet wird. Vorzugsweise liegt der Innenschicht derselbe synthetischen Latex wie der Außenschicht zugrunde.

Nach einer Ausgestaltung ist der synthetische Latex Polyisopren, vorzugsweise 1,4-cis-Polyisopren.

Nach einer Ausgestaltung umfasst das Kondom ein Trennmittel, das das Ablösen von einem Formkörper bei der Herstellung bzw. die Benutzung erleichtert.

Ferner wird die Aufgabe gelöst durch ein Verfahren zum Herstellen von Kondomen der vorerwähnten Art, bei dem
- ein Formkörper mit der Form eines Kondoms in eine erste Mischung enthaltend einen synthetischen Latex und einen Vulkanisator getaucht und der Formkörper aus der ersten Mischung unter Mitnahme eines ersten Filmes zum Bilden einer Innenschicht herausgezogen wird,
- danach der Formkörper in eine zweite Mischung enthaltend einen Naturkautschuklatex und einen Vulkanisator getaucht und der Formkörper unter Mitnahme eines zweiten Filmes zum Bilden einer Zwischenschicht aus der zweiten Mischung herausgezogen wird und
- danach der Formkörper in eine dritte Mischung enthaltend einen synthetischen Latex und einen Vulkanisator getaucht und unter Mitnahme eines äußeren Filmes zum Bilden einer Außenschicht aus der dritten Mischung herausgezogen wird.

In der vorbeschriebenen Weise kann das Sandwichkondom einfach in einem kontinuierlichen Verfahren aus einer mit drei Tauchbecken ausgerüsteten Tauchkette bekannter Bauart hergestellt werden oder im Batchtauchverfahren mit drei Tauchbecken oder austauschbaren Tauchbecken, an Tauchgeräten oder Tauchketten, wie sie in der Handschuhherstellung oder der Medizinprodukteherstellung genutzt werden. Die Mischungen werden so zusammengesetzt, dass sie im jeweils angewandten Produktionsverfahren eingesetzt werden können. Der Vulkanisator kann so ausgewählt werden, dass die Schichten unter Wärmeeinfluss vulkanisieren.

Beim kontinuierlichen Verfahren sind die Formkörper auf einer endlosen Kette befestigt, die die Produktionsschritte kontinuierlich durchfährt. Dies bedingt eine ununterbrochene horizontale Bewegung der Formkörper, auch beim Tauchvorgang. Damit die Formkörper das Tauchbad nicht durchfurchen müssen, was zu einer Bugwelle an jedem Formkörper und letztendlich zu starken Wanddickenschwankungen führen würde, wird die Mischung gemäß einer Ausgestaltung des Verfahrens in speziell hierzu konstruierten Tauchbecken mittels Rührwerken in eine Fließbewegung versetzt. Diese wird so angepaßt, daß sich die horizontalen Bewegungskomponenten genau überlappen und relativ gesehen nur eine vertikale Austauchbewegung übrig bleibt. Der Vorteil des kontinuierlichen Verfahrens gegenüber dem diskontinuierlichen Verfahren ist ein optimaleres Verhältnis zwischen Investitionskosten und Produktionsausstoß.

Grundsätzlich können die Latices für die verschiedenen Schichten verschiedene oder übereinstimmende Vulkanisatoren enthalten. Gemäß einer Ausgestaltung ist der Vulkanisator Schwefel. Gemäß einer weiteren Ausgestaltung enthält die erste und/oder dritte Mischung 0,3 bis 0,5 phr Schwefel und/oder die zweite Mischung 0,65 bis 1,0 phr Schwefel. Mit "phr" sind die Gewichtsteile bezogen auf 100 Gewichtsteile Kautschuk bezeichnet.

Gemäß einer Ausgestaltung enthält die erste und/oder zweite und/oder dritte Mischung einen Vulkanisationsbeschleuniger. Gemäß einer weiteren Ausgestaltung ist der Vulkanisationsbeschleuniger ein Dithiocarbamat, vorzugsweise Dialkyldithiocarbamat oder MBT (Merkaptobenzothiazol). Gemäß einer weiteren Ausgestaltung enthält die erste und/oder dritte Mischung 0,2 bis 0,5 phr und/oder die zweite Mischung 0,5 bis 1,0 phr Vulkanisationsbeschleuniger.

Gemäß einer Ausgestaltung enthält die erste und/oder zweite und/oder dritte Mischung einen Aktivator. Aktivatoren sind Vulkanisationshilfsmittel, die o.g. Vulkanisationsbeschleuniger aktivieren. Gemäß einer weiteren Ausgestaltung ist der Aktivator ZnO. Gemäß einer weiteren Ausgestaltung enthält die erste und/oder dritte Mischung 0,0 bis 0,5 phr Aktivator und/oder zweite Mischung 0,65 bis 1,0 phr Aktivator.

Gemäß einer Ausgestaltung enthält die erste und/oder zweite und/oder dritte Mischung einen Stabilisator zur Stabilisierung der Latex-Dispersion. Gemäß einer weiteren Ausgestaltung enthält die erste und/oder zweite und/oder dritte Mischung 0,0 bis 1,0 phr Stabilisator.

Gemäß einer Ausgestaltung hat die erste und/oder zweite und/oder dritte Mischung eine Temperatur von 5 bis 35°C, je nach der gewünschten Dicke des Films, der von dem Formkörper aufgenommen wird. Mit steigender Temperatur der Mischung sinkt die Dicke des aufgenommenen Filmes.

Beim "Mischen" wird den Latexdispensionen die nötige Menge an Vulkanisationschemikalien zugesetzt, um eine ausreichende "Vorvernetzung" zu gewährleisten. Der dabei erreichte Vernetzungsgrad ist aber noch nicht ganz der im Zuge des Trocknens der getauchten Kondome erreichte Endvernetzungsgrad.

Die Verfestigung der Schichten erfolgt im Wesentlichen nach Entnahme des Formkörpers aus den Mischungen. Dies geschieht beim Austauchen durch Trocknen der Filme und Diffusion von Molekülen im Grenzschichtbereich (Interdiffüsion). Ferner durch Vulkanisation der Filme. Hierzu werden die Filme mit einer Temperatur zwischen 40 und 120°C aufgeheizt. Bevorzugt wird eine Heizeinrichtung verwendet. Vorzugsweise werden die Kondome mittels Heißluft aufgeheizt. Bei einer Temperatur von ca. 70°C sind die Kondome nach ca. 10 bis 30 Minuten hinreichend vulkanisiert und weiterverarbeitbar. Durch Erwärmung mittels Heißluft wird zugleich ein Trocknen des Kondoms erreicht.

Bereits beim Tauchen findet im Bereich der Grenzschichten zwischen den verschiedenen Filmen von beiden Seiten eine gegenseitige Durchdringung auf molekularer Ebene statt. Gemäß einer Ausgestaltung wird dies durch Einmischen von etwas synthetischen Latex in die zweite Mischung und damit in den mittleren Film gefördert. Im weiteren Vulkanisationsprozess wird diese Durchdringung (Interdiffusion) durch geringfügige Vernetzung zusätzlich mechanisch verstärkt. Grundsätzlich reicht jedoch schon die Interdiffüsion aus, die Schichten des Kondoms hinreichend miteinander zu verbinden.

Gemäß einer Ausgestaltung wird das Kondom nach dem Vulkanisieren bzw. Trocknen vom Formkörper abgerollt. Gemäß weiteren Ausgestaltungen geschieht dies mittels rotierender Rundbürsten und/oder mittels eines Flüssigkeitsstrahls. Danach können sie verpackt werden.

Gemäß einer weiteren Ausgestaltung wird der Formkörper mit dem darauf befindlichen Kondom vor dem Abrollen in ein Einweichbad getaucht, um das Abrollen zu erleichtern. Gemäß einer weiteren Ausgestaltung ist das Einweichmittel ein Trennmittel.

Nachfolgend wird die Erfindung anhand von Beispielen für Rezepturen und die Verfahrensdurchführung näher erläutert:

### Rezepturen für Polyisoprenlatex und Naturkautschuklatex:

Hauptbestandteil der ersten Mischung ist ein rein synthetischer Latex aus unvernetztem 1.4-cis-Polyisopren (beispielsweise Kraton-IR-Typ). Die Teilchengrößen des synthetischen Latex können bei breiter Verteilung zwischen 20 und 100 µm variieren, der Feststoffgehalt des synthetischen Latex kann zwischen 45 und 65 Gew.-% liegen, wobei höhere Feststoffgehalte bevorzugt werden.

Hauptbestandteil der zweiten Mischung ist unvernetzter HA-Naturkautschuklatex.

HA - Naturkautschuklatex ist "high ammonia latex". Grundsätzlich kann auch low ammonia latex oder sogar ultralow ammonia latex zur Anwendung kommen, wobei man entsprechend zusätzlich stabilisieren muß.

Die dritte Mischung entspricht der ersten Mischung.

Vor dem Mischen wird der synthetische bzw. Naturkautschuklatex zusätzlich kolloidal stabilisiert, wobei Alkalien, ionische oder nichtionische Seifen oder deren Kombinationen eingesetzt werden können.

Hiernach werden die entsprechenden Vulkanisatoren als Dispersion sukzessive oder in Form eines Chemikalienansatzes sehr langsam zugesetzt, wobei ständig schonend gerührt wird. In großtechnischem Maßstab wird bevorzugt mit einem Chemikalienansatz gearbeitet, dessen Zusammensetzung und Menge dem Latex sowie der folgenden Kondomproduktion angepasst werden muss. Es wird eine zinkaktivierte, dithiocarbamatbeschleunigte oder MBT-beschleunigte Schwefelvulkanisation durchgeführt. Die Rezeptur ist die im Allgemeinen für die Produktion von Kondomen verwendete, angepasst an die jeweiligen Stabilitätsverhältnisse, mechanischen Anforderungen und unterschiedlichen Vulkanisationsgeschwindigkeiten:

### Rezepturen (Angaben in phr):

| | Mischung 1: | Mischung 2: |
|---|---|---|
| Vulkanisator Schwefel: | 0,3 bis 0,5 | 0,65 bis 1,0 |
| Aktivator ZnO: | 0,0 bis 0,5 | 0,65 bis 1,0 |
| Beschleuniger Dialkyldithiocarbamat: | 0,2 bis 0, 5 | 0,5 bis 1,0 |
| ASM: | 0,0 bis 0,5 | 0,0 bis 0,75 |
| Kolloidale Stabilisatoren: | 0,0 bis 1,0 | 0,0 bis 1,0 |

"ASM" bezeichnet ein Alterungsschutzmittel. Dieses verbindet eine vorzeitige Alterung durch "Abfangen" von Oxidationsprozessen.

Dabei ist es wichtig, einen bestimmten Vorvulkanisationsgrad zu erreichen, der die Fließfähigkeit des Polyisoprens in genügendem Maße einschränkt, sodass gummiähnliche Eigenschaften schon im Bereich der Ränderstation (siehe unten) vorliegen. Zu diesem Zwecke wird der Latex nach Zugabe der Chemikalien bei erhöhter Temperatur (26 bis 75°C) über einen bestimmten Zeitraum (1 bis 30 Tage) gereift. Andererseits darf der Vorvernetzungsgrad eine obere Grenze nicht überschreiten, bei der gummiähnliche Eigenschaften der Latexteilchen eine Filmbildung unterbinden. Hierzu kann entweder die Chemikalienzusammensetzung so gewählt werden, dass der gewünschte Vorvernetzungsgrad bei maximaler Umsetzung gerade erreicht wird, oder die Vorvernetzung wird in Gegenwart eines Chemikalienüberschusses durchgeführt und nach Erreichen des Optimums durch Abkühlen so weit verlangsamt, dass die Produzierfähigkeit erhalten bleibt.

Prinzipiell ist zu berücksichtigen, dass die Stabilität des synthetischen Latex eine andere ist als die von Naturlatex. Aus diesem Grunde kann nach der Chemikalienzugabe nochmals stabilisiert werden, wobei wiederum Alkalien, ionische oder nichtionische Seifen oder deren Kombination eingesetzt werden können. Ebenso muss die Zugabe des Aktivators an die Stabilitätsverhältnisse angepasst werden.

Die gängigen Standardmethoden zur Beurteilung des Vorvernetzungsgrades können herangezogen werden. Der optimale Vorvernetzungsgrad ist dann erreicht, wenn dünne, getrocknete Filme einen geringen Tack zeigen, aber noch transparent sind. "Tack" bezeichnet die Klebrigkeit bei kurzem Kontakt.

Zur Optimierung der Produktionseigenschaften können die Mischungen mit weiteren Agenzien versetzt werden, wie z.B. synthetische wasserlösliche Polymere, Oligomere oder Mineralien. Die so hergestellten Mischungen haben Feststoffgehalte zwischen 45 und 65 Gew.-% und können für sich genommen auf einer herkömmlichen Produktionsanlage produziert werden.

### Tauchen des Sandwichkondoms:

### Prinzip:

Bei dem erfindungsgemäßen Verfahren werden drei Tauchschritte durchgeführt. Zunächst wird der Formkörper in die erste Mischung getaucht und nach dem Herausziehen der darauf verbleibende Film getrocknet. Es folgt ein verstärkender Tauch mit der zweiten Mischung. Zur Vermeidung von Benetzungsproblemen wird die zweite Mischung bevorzugt mit 5 bis 30 % der ersten Mischung verschnitten. Der gut aufgetrocknete zweite Film wird dann nochmals mit der ersten Mischung übergetaucht.

### Temperatur und Zeiten im Tauchbad:

Tauchbäder aus der ersten bis dritten Mischung haben gleichermaßen Temperaturen im Bereich von 5 bis 35°C, je nach der gewünschten Stärke des vom Formkörper aufgenommenen Films. Der Vorgang ist weitgehend unabhängig von den Eintauchzeiten. Die Austauchgeschwindigkeit beträgt bevorzugt 0,5 bis 1,5 cm/sec. Bevorzugt ist die Tauchtiefe des zweiten Tauchgangs geringer als die des ersten und des dritten Tauchganges. Hierdurch wird eine direkte Verbindung der Innenschicht und der Außenschicht am Öffnungsrand des Kondoms erreicht und der Naturkautschuklatex vollständig eingekapselt.

### Fließgeschwindigkeit:

Die Fließgeschwindigkeit der Mischungen ist beim Batchverfahren nicht relevant. Bei Anwendung einer Tauchkette ist die Fließgeschwindigkeit der Kettengeschwindigkeit angepasst.

### Verweildauer:

Die Verweildauer des Formkörpers in den verschiedenen Tauchbädern ist möglichst kurz gehalten, um einen möglichst hohen Durchsatz zu erzielen. Die Austauchgeschwindigkeit wird durch Anstellwinkel und Kettengeschwindigkeit eingestellt und beeinflußt die ausgetauchte Schichtdicke.

### Vulkanisationstemperatur:

Temperaturen zwischen 40 und 120°C sind möglich, mit variabler Belüftung. Üblicherweise führen Temperaturen von ca. 70°C während einer Vulkanisationsdauer von 10 bis 30 Minuten zu weiterverarbeitbarer Ware.

### Vernetzung:

Im Bereich der Grenzschicht zwischen den verschiedenen Schichten findet von beiden Seiten eine gegenseitige Durchdringung auf molekularer Ebene statt, die durch das Einmischen von etwas synthetischem Latex in die mittlere Schicht aus Naturkautschuklatex erleichtert wird. Im weiteren Vulkanisationsprozess wird diese Durchdringung durch geringfügige Vernetzung zusätzlich mechanisch verstärkt.

### Aggregatzustand:

Die synthetischen bzw. Naturkautschuklatex enthaltenden Mischungen sind im flüssigen Aggregatzustand. Getrocknet bzw. ausvulkanisiert ist der Aggregatzustand fest bzw. gummiartig.

### Natürliche Nachvulkanisation:

Eine natürliche Nachvulkanisation findet statt und das Alterungsverhalten des Produktes ist sehr gut.

### Abziehverhalten von den Formkörpern:

Nach dem letzten Tauchgang wird auf einer weiteren Trockenstation getrocknet, bis die übereinandergeschichteten Filme ränderfähig sind. Die Bedingungen ähneln denen im ersten Trockenbereich. Ränderfähig heißt, dass das Trocknen und die Koaleszenz des Latexfilms gerade so weit fortgeschritten sind, dass der Film sich von der Form abrollen lässt und nicht zerreißt, und dass andererseits die Vulkanisation noch nicht weit genug fortgeschritten ist, um eine Koaleszenz (Interdiffüsion) der gerollten Schichten zu verhindern.

Das Rändern findet wie üblich unter Rotieren der Formkörper mit einer angestellten, schnelldrehenden Ränderbürste (beading round brush) statt.

Für die Weiterverarbeitung der Ware ist es wichtig, die geformten Kondome unversehrt von dem Formkörper abzustreifen. Ein Quellen des Kondoms auf dem Formkörper findet nicht statt. Vielmehr wird das auf dem Formkörper getrocknete Kondom in ein Einweichbecken mit einer Lösung aus Alkalien oder nichtionischen Seifen oder deren Kombination eingetaucht, die zwischen dem Formkörper (z.B. aus Glas) und das Kondom kriecht. Hierdurch wird das leichte Abstreifen oder Abspritzen des Kondoms von dem Formkörper ermöglicht und ein nachfolgendes Verkleben verhindert.

Direkt nach dem Einweichbad gelangt der getauchte Formkörper an eine Abspritz- oder Abbürststation, wo die Kondome mit Hilfe von angestellten rotierenden Rundbürsten oder eine Flüssigkeitsstrahls oder durch die Kombination von beiden von dem Formkörper entfernt werden. Hierbei ist es wichtig, das Kondom mit einer Detergenzienlösung, die identisch mit der Einweichbeckenbefüllung sein kann, reichlich zu befeuchten, da sonst ein Verkleben eintritt. Es ist auch möglich, das Kondom sofort mit einer Trennmitteldispersion zu behandeln.

### Allgemeines zu den Begriffen Vorvernetzung , Vernetzung, Trocknung, Vulkanisation, Rollen des Rollrandes und Abziehen:

Bei Latices versteht man unter Vorvemetzung die Vernetzung innerhalb der individuellen Latexteilchen. Bei den heutigen Kondomproduktionen ist zumeist der Grad der Vorvemetzung im wesentlichen schon der Endvernetzungsgrad. Dies wird während der Reifung der Mischungen durch den Einsatz von sehr aktiven Beschleunigern erreicht.

Nach dem Austauchen der Formkörper trocknet die Schicht aus Latex durch Verdunsten von Wasser. Hierbei soll es zur Filmbildung kommen, d.h., die Teilchen müssen sich noch plastisch deformieren (zur Erreichung eines "kohärenten Films") und die Polyisoprenmoleküle sollen noch durch die Grenzschicht zwischen den ehemals individuellen Teilchen diffundieren können (Interdiffusion). Dies bewirkt den materiellen Zusammenhalt und damit die Festigkeit des Sandwich-Kondoms. Ist der Vorvernetzungsgrad zu hoch, so können sich die Teilchen nicht mehr plastisch deformieren und Molekülketten nicht mehr genügend weit diffundieren, so dass die getrocknete Schicht Hohlräume enthält und brüchig ist.

Da die Chemikalien nach wie vor im Material sind, kommt es im Film, beschleunigt durch Wärmezufuhr, zu einer weiteren Vernetzung, welche die Endfestigkeit erhöht. Wäre diese im Bereich der Ränderstation schon zu weit fortgeschritten, so käme es zu keiner "Filmbildung" zwischen den gerollten Schichten mehr. Ist die Mischung zu frisch gewesen, so klebt der Kautschukfilm so stark an den Formkörpem, dass es Schwierigkeiten beim Rollen und Abziehen gibt.

Ist die Mischung zu reif, wird die Ware nicht fest genug und reißt beim Rändern. Ist das Kondom beim Abziehen nicht genug vulkanisiert, so ist des Tack so stark, dass alle Kondome verkleben (trotz Trennmittel). Ist es übervulkanisiert, so sind die Alterungswerte schlecht.

Durch die Variation von Temperatur und Belüftung in den Heizzonen wird Einfluss auf die Filmbildung und Endvulkanisation genommen, um ein optimales Ergebnis zu erzielen.

## Patentansprüche

1. Kondom umfassend von innen nach außen
1.1 eine Innenschicht hergestellt aus einer synthetischen Latex enthaltenden Zusammensetzung,
1.2 eine Zwischenschicht hergestellt aus einer Naturkautschuklatex enthaltenden Zusammensetzung und
1.3 eine Außenschicht hergestellt aus einer synthetischen Latex enthaltenden Zusammensetzung.

2. Kondom nach Anspruch 1, bei dem die Innenschicht und die Zwischenschicht und/oder die Zwischenschicht und die Außenschicht einander im Grenzbereich durchdringen und/oder miteinander im Grenzbereich vernetzt sind.

3. Kondom nach Anspruch 1 oder 2, bei dem die Innenschicht und/oder die Außenschicht aus einer im Wesentlichen synthetischen Latex enthaltenden Zusammensetzung hergestellt ist.

4. Kondom nach einem der Ansprüche 1 bis 3, bei dem die Zwischenschicht aus einer synthetischen Latex enthaltenden Zusammensetzung hergestellt ist.

5. Kondom nach Anspruch 4, bei dem die Zwischenschicht aus einer 20 bis 30 Gew.-% synthetischen Latex enthaltenden Zusammensetzung hergestellt ist.

6. Kondom nach einem der Ansprüche 1 bis 5, bei dem der synthetische Kautschuk Polyisopren ist.

7. Kondom nach Anspruch 6, bei dem der synthetische Kautschuk 1.4-cis-Polyisopren ist.

8. Kondom nach einem der Ansprüche 1 bis 7, das ein Trennmittel umfasst.

9. Kondom nach einem der Ansprüche 1 bis 9, bei dem die Innenschicht und die Außenschicht am Öffhungsrand miteinander verbunden sind.

10. Verfahren, insbesondere zum Herstellen von Kondomen nach einem der Ansprüche 1 bis 9, bei dem
10.1 ein Formkörper mit der Form eines Kondoms in eine erste Mischung enthaltend einen synthetischen Latex und einen Vulkanisator getaucht wird und der Formkörper aus der ersten Mischung unter Mitnahme eines ersten Filmes zum Bilden einer Innenschicht herausgezogen wird,
10.2 danach der Formkörper in eine zweite Mischung enthaltend einen Naturkautschuklatex und einen Vulkanisator getaucht wird und der Formkörper unter Mitnahme eines zweiten Filmes zum Bilden einer Zwischenschicht aus der zweiten Mischung herausgezogen wird und
10.3 danach der Formkörper in eine dritte Mischung enthaltend einen synthetischen Latex und einen Vulkanisator getaucht und unter Mitnahme eines dritten Filmes zum Bilden einer Außenschicht aus der dritten Mischung herausgezogen wird.

11. Verfahren nach Anspruch 10, bei dem der Vulkanisator Schwefel enthält.

12. Verfahren nach Anspruch 11, bei dem die erste und/oder die dritte Mischung 0,3 bis 0,5 phr Schwefel und/oder die zweite Mischung 0,65 bis 1,0 phr Schwefel enthält/enthalten.

13. Verfahren nach einem der Ansprüche 10 bis 12, bei dem die erste und/oder zweite und/oder dritte Mischung einen Aktivator enthält.

14. Verfahren nach Anspruch 13, bei dem der Aktivator Zinkoxid ist.

15. Verfahren nach Anspruch 13 oder 14, bei dem die erste und/oder dritte Mischung 0,0 bis 0,5 phr Aktivator und/oder die zweite Mischung 0,65 bis 1,0 phr Aktivator enthält.

16. Verfahren nach einem der Ansprüche 10 bis 15, bei dem die erste und/oder zweite und/oder dritte Mischung einen Vulkanisationsbeschleuniger enthält.

17. Verfahren nach Anspruch 16, bei dem der Vulkanisationsbeschleuniger ein Dithiocarbamat oder MBT ist.

18. Verfahren nach Anspruch 16 oder 17, bei dem die erste und/oder dritte Mischung 0,2 bis 0,5 phr Vulkanisationsbeschleuniger und/oder die zweite Mischung 0,5 bis 1,0 phr Vulkanisationsbeschleuniger enthält.

19. Verfahren nach einem der Ansprüche 10 bis 18, bei dem die erste und/oder zweite und/oder dritte Mischung einen Stabilisator enthält.

20. Verfahren nach Anspruch 19, bei dem der erste und/oder zweite und/oder dritte Mischung 0,0 bis 1,0 phr Stabilisator enthält.

21. Verfahren nach einem der Ansprüche 10 bis 20, bei dem die erste und/oder zweite und/oder dritte Mischung eine Temperatur von 5 bis 35°C aufweist.

22. Verfahren nach einem der Ansprüche 10 bis 21, bei dem die übereinandergelagerten Filme bei einer Temperatur zwischen 40 und 120°C vulkanisiert werden.

23. Verfahren nach einem der Ansprüche 10 bis 22, bei dem in die zweite Mischung ein Teil der ersten und/oder der dritten Mischung eingemischt wird.

24. Verfahren nach einem der Ansprüche 10 bis 23, bei dem der Formkörper tiefer in die erste und dritte Mischung eingetaucht wird als in die zweite Mischung.
